Europäisches Patentamt

European Patent Office

Office européen des brevets

(11) Numéro de publication: **0 156 691**

**A1**

(12) # DEMANDE DE BREVET EUROPEEN

(21) Numéro de dépôt: **85400354.8**

(22) Date de dépôt: **26.02.85**

(51) Int. Cl.⁴: **B 01 J 23/76**
**C 07 C 29/15**

(30) Priorité: **13.03.84 FR 8403806**

(43) Date de publication de la demande:
**02.10.85 Bulletin 85/40**

(84) Etats contractants désignés:
**AT BE CH DE FR GB IT LI LU NL SE**

(71) Demandeur: **SOCIETE CHIMIQUE DE LA GRANDE PAROISSE, AZOTE ET PRODUITS CHIMIQUES**
**8, rue Cognacq-Jay**
**F-75007 Paris(FR)**

(72) Inventeur: **Denise, Bernard**
**32, avenue du Mont Blanc**
**F-69140 Rillieux(FR)**

(72) Inventeur: **Sneeden, Raymond**
**13, avenue des Armières**
**F-38290 Villefontaine(FR)**

(72) Inventeur: **Hamon, Christian**
**47, Chemin des Dames**
**F-44600 Saint Nazaire(FR)**

(74) Mandataire: **Bouton Neuvy, Liliane et al,**
**L'Air liquide, Société Anonyme pour L'Etude et L'Exploitation des Procédés Georges Claude 75, Quai d'Orsay**
**F-75321 Paris Cedex 07(FR)**

(54) **Méthode de préparation de catalyseurs de synthèse du méthanol, et catalyseurs obtenus.**

(57) L'invention concerne une méthode de préparation de catalyseurs à base de cuivre associé à des supports minéraux, choisis parmi les oxydes des métaux de la famille des alcalino-terreux, des lanthanides ou des actinides.

Le cuivre est introduit dans le catalyseur à partir d'un précurseur de formiate de cuivre. Le mode de préparation de ces masses de contact consiste en l'imprégnation du support, suivi d'une thermodécomposition sous hydrogène.

Ces catalyseurs sont très performants dans la synthèse du méthanol, notamment à partir de mélange monoxyde de carbone — hydrogène.

EP 0 156 691 A1

CATALYSEURS OBTENUS".

0156691

La présente invention, réalisée avec la coopération du CNRS (Institut de Recherches sur la Catalyse), concerne une méthode de préparation de catalyseurs à base de cuivre, en vue de leur application dans la synthèse du méthanol à partir d'oxydes de carbone et d'hydrogène.

Anciennement, les catalyseurs conventionnels étaient à base d'oxyde de zinc dopé à l'oxyde de chrome $Cr_2O_3$ pour les procédés fonctionnant à haute pression, supérieure à 100 bars.

Dans les procédés basse pression, voisine de 50 bars, concernant actuellement l'essentiel des unités existantes, les catalyseurs se composent d'un mélange d'oxyde de zinc et d'oxyde de cuivre ; ce dernier étant préalablement réduit par un courant d'hydrogène dilué par de l'azote avant la mise en oeuvre du catalyseur. Ces catalyseurs cuivre-zinc sont préparés par précipitation contrôlée des carbonates correspondants, à partir d'une solution des nitrates, et ils renferment d'importantes quantités de cuivre, de l'ordre de 35 à 65 % CuO.

De nouvelles méthodes de préparation de catalyseurs cuivre-zinc ont été décrites. Elles concernent plus particulièrement le choix des précurseurs du cuivre. L'utilisation de carboxylates a été signalée, et le passage par des intermédiaires citrates a fait l'objet du brevet d'invention 2.529.098.

De nouveaux catalyseurs, également à base de cuivre, ont été proposés, le cuivre étant associé à des oxydes de métaux de la famille des lanthanides ou des actinides. Le brevet américain 4.181.630, décrit la préparation de catalyseurs pour la synthèse du méthanol par oxydation d'alliages associant le cuivre aux métaux précités. Des catalyseurs cuivre-thorine issus d'alliages Cu/Th apparaissent comme les plus performants.

La préparation de ce type de catalyseur ($Cu/ThO_2$) a également été réalisée en utilisant la technique d'échange cationique pour la fixation du cuivre sur la thorine. L'intérêt de ces catalyseurs réside dans leur activité élevée pour la production de méthanol, à partir de mélanges monoxyde de carbone-hydrogène ; celle-ci est supérieure à celle des catalyseurs conventionnels et ceci pour des teneurs en cuivre plus faibles.

L'objet de l'invention concerne une méthode d'obtention de cette nouvelle génération de catalyseurs associant le cuivre à des supports minéraux. Ceux-ci ont été choisis parmi les oxydes des métaux de la famille des alcalino-terreux, des lanthanides ou des actinides.

0156691

Il a été découvert que la nature des précurseurs du cuivre joue un rôle déterminant dans l'activité du catalyseur dans la synthèse du méthanol. Ainsi, l'utilisation de formiates de cuivre pour l'imprégnation des supports constitués par les oxydes des métaux de la famille des alcalino-terreux, des lanthanides et des actinides, conduit à des catalyseurs plus performants que ceux obtenus par la même technique à partir d'autres sels, tels nitrates ou carboxylates (J.Saussey et al J. Chem. Soc. Chem. Comm. 1982 p.278).

La nature du support est aussi très importante ; la présence d'un élément basique ou d'un composant basique présente un avantage certain, on peut citer l'oxyde de magnésium, la magnésie, MgO et l'oxyde de thorium, la thorine $ThO_2$.

La préparation des catalyseurs est effectuée par imprégnation du support, cette technique connue en elle-même, offre l'avantage de ne pas être limitative quant à la teneur en cuivre déposé, ceci contrairement par exemple à la technique par échange cationique (E.Druet et al 8ème colloque Franco-polonais Poitiers, septembre 1981)

Le précurseur est dissous dans l'eau, à cette solution on ajoute en suspension l'oxyde métallique constituant le support, après une durée d'imprégnation de l'ordre de quelques minutes, comprise entre 1 et 15 minutes, la solution est évaporée sous vide, dans des conditions douces, à température comprise entre 60 et 95°C, jusqu'à siccité et le solide est soumis à un broyage.

Ensuite, la masse catalytique est thermodécomposée sous courant d'hydrogène ; cette opération de réduction sous hydrogène est conduite à une température comprise entre 250 et 400°C, de préférence 300°C, pendant une durée supérieure à 1 heure, de préférence 6 heures, après une montée en température avec un gradient de 1°C $mn^{-1}$, avec un débit d'hydrogène, exprimé en vitesse volumétrique compris entre environ 5.000 à 50.000 $h^{-1}$. Ce débit d'hydrogène est compris entre 1 et 10 1 Nl/h de préférence 2,5 Nl/h pour une masse de catalyseur de 200mg Le gradient de montée en température a une incidence sur l'activité catalytique, en le multipliant par 3, soit 3 $mn^{-1}$, on a noté sur les catalyseurs ainsi préparés une perte d'activité d'environ 20 % pour la synthèse du méthanol.

Avec des supports oxydes purs, la quantité efficace de cuivre varie selon la nature de cet oxyde, avec la thorine ($ThO_2$) l'opti-

3

mum cuivre se situe entre 0,5 et 10 % en poids, par rapport au catalyseur réduit, de préférence entre 1 à 5 %, avec la magnésie (MgO),
cette teneur en cuivre se situe entre 10 et 50 % en poids par rapport
au catalyseur réduit, de préférence entre 15 et 25 %.

Les résultats les plus performants sont constatés avec
l'association du choix de la nature du précurseur : formiate de cuivre, à la technique de fabrication par imprégnation du support par le
précurseur du cuivre suivie d'une thermodécomposition sous gaz : hydrogène ..

Les catalyseurs préparés selon la méthode de l'invention
se différencient également des catalyseurs dits classiques "Cu/Zn" au
niveau de leur mise en oeuvre dans la synthèse du méthanol. Dans le
cas des catalyseurs au cuivre obtenus à partir de formiate de cuivre
il n'est pas nécessaire d'opérer en présence de dioxyde de carbone
dans le gaz de synthèse, alors que celui-ci est indispensable pour
les catalyseurs classiques, au minimum quelques pourcents de $CO_2$ dans
le mélange entrant.

Les catalyseurs préparés selon la technique décrite sont
très performants dans la synthèse du méthanol à partir de mélange
monoxyde de carbone-hydrogène.

Il est donné ci-après quelques exemples de la méthode de
préparation des catalyseurs et de leur application dans la synthèse
du méthanol à partir de monoxyde de carbone et d'hydrogène.

Exemple 1. On dissout 480 mg de formiate de cuivre valence
2, de formule $(HCO_2)_2Cu$ dans 20 ml d'eau, puis ajoute en suspension
108 mg d'oxyde de thorium $(ThO_2)$ —celui-ci a été préparé par calcination sous air à 500°C du nitrate de thorium— la durée d'imprégnation
est de 5 minutes. Ensuite, on évapore la solution sous vide (15 à 20
mbars) à 72°C, pendant une heure, à l'évaporateur rotatif. Le solide
est alors broyé, on récupère 250 mg de catalyseur, qui sont placés
dans un microréacteur en quartz.

On réduit le catalyseur sous courant d'hydrogène 2,5 $Nlh^{-1}$
à 300°C pendant six heures, après une montée en température de 1°C$mn^{-1}$
puis soumet le catalyseur réduit en régime dynamique au test catalytique pour la transformation de mélange monoxyde de carbone et hydrogène, dans les proportions $1CO - 3H_2$. On effectue ces essais à pression atmosphérique à une température voisine de 250°C ; le débit du
mélange hydrogène - CO est de 1,2 Nl/h.

On a préparé selon le mode opératoire décrit ci-dessus

4    **0156691**

différents échantillons de catalyseurs en faisant varier la nature du support. La teneur en cuivre des échantillons est voisine de 25 % sur le catalyseur non réduit.

On conduit les essais comparatifs sur une masse de 200 mg de catalyseur réduit, les différents échantillons et les catalyseurs sont testés dans les mêmes conditions, ainsi que sur un catalyseur industriel de synthèse du méthanol de composition CuO = 46, ZnO = 23. Les quantités de produits formés : méthanol ($CH_3OH$), dioxyde de carbone ($CO_2$) et hydrocarbures sont exprimées en p.p.m.

TABLEAU I

| Nature du support | $CH_3OH$ | $CO_2$ | Hydrocarbures |
|---|---|---|---|
| ZnO | 34 | 2000 | 2 |
| MgO | 283 | 2000 | 20 |
| CaO | 86 | 440 | 3 |
| $ThO_2$ | 63 | 1420 | 3 |
| $Nd_2O_3$ | 163 | 325 | 1 |
| Cu - Zn industriel | 160 | 2100 | 1 |
| CoO | 0 | 200 | 40 |

Dans les conditions de l'essai, le palier thermodynamique se situe vers 280 p.p.m.

Exemple 2. On réalise différentes préparations de catalyseurs selon la technique de l'exemple 1 en faisant varier la teneur en cuivre et ceci avec deux supports : la thorine et la magnésie. Ces catalyseurs sont testés à l'état non réduit sur 200 mg, dans les mêmes conditions que précédemment à savoir en régime dynamique avec un mélange monoxyde de carbone-hydrogène $H_2/CO = 3$, de débit 1,2 Nl/heure, sous la pression atmosphérique, à température de 250°C.

Les résultats obtenus sont consignés dans le tableau ci-après.

TABLEAU II

| Nature du support | Teneur en Cuivre (avant réduction) | $CH_3OH$   p.p.m |
|---|---|---|
| $ThO_2$ | 28 | 42 |
|  | 26,4 | 140 |
|  | 13,8 | 262 |
|  | 7,4 | 290 |
|  | 2,4 | 296 |
| MgO | 33,2 | 110 |
|  | 25,8 | 242 |
|  | 15,9 | 240 |
|  | 5,6 | 200 |

Avec un catalyseur dont le support est constitué par la thorine, avec des teneurs en cuivre de 7,4 et 2,4 % on atteint la limitation thermodynamique dans les conditions réactionnelles de l'essai. D'autre part, les productions de méthanol différentes dans les premier et deuxième exemples à teneur égale en cuivre, voisine de 25 %, sont la conséquence de la conduite des essais sur des masses de catalyseurs différentes, dans un cas il s'agit de 200 mg avant réduction dans l'autre après thermodécomposition – réduction.

On constate que l'activité du catalyseur cuivre associé à la thorine augmente lorsque la teneur en cuivre diminue. Elle est maximale pour seulement quelques pourcents de cuivre (environ 5 %). Et sa productivité est alors nettement supérieure à celle des catalyseurs classiques.

Le phénomène se retrouve avec la magnésie tout en étant plus atténué ; la productivité des catalyseurs Cu/MgO reste sensiblement constante dans un large domaine de concentration en cuivre.

Exemple 3. Etude de l'influence de la nature du précurseur.

Selon la même technique de l'invention, par imprégnation suivie d'une thermodécomposition sous hydrogène, on prépare trois échantillons de catalyseurs Cu/MgO à teneur identique, 25 % de cuivre avant réduction. Le premier catalyseur est obtenu à partir de formiate de cuivre II, suivant la méthode décrite dans l'exemple 1, et les deux autres catalyseurs respectivement à partir d'acétate de cuivre II $(CH_3 CO_2)_2$ Cu et de nitrate du cuivre.

Ces trois échantillons de catalyseur sont soumis au test catalytique dans des conditions identiques à celles de l'exemple 1, chaque fois à partir de 200 mg de catalyseur réduit. Les résultats obtenus sont consignés dans le tableau ci-dessous.

TABLEAU III

| Nature du précurseur | $CH_3OH$ p.p.m | $CO_2$ |
|---|---|---|
| Formiate | 283 | 2000 |
| Acétate | 77 | 420 |
| Nitrate | 70 | 1100 |

Avec le formiate de cuivre comme précurseur, on atteint le palier thermodynamique dans les conditions de l'essai.

La lecture des résultats met en évidence l'importance de la nature du précurseur du cuivre, et fait apparaitre le très net avantage du formiate de cuivre par rapport aux nitrate acétate.

Exemple 4. Etude de l'influence de la méthode de prépara-

tion du catalyseur, avec une teneur en cuivre identique et même nature de support.

On obtient le premier catalyseur par mélange intime de 10 mg de formiate de cuivre avec 190 mg de magnésie (MgO).

Le second catalyseur de même composition est préparé par imprégnation selon la méthode décrite dans l'exemple 1.

La teneur en cuivre de ces deux catalyseurs est voisine de 2 %, avant leur soumission à une thermodécomposition sous hydrogène.

Les résultats obtenus dans la transformation de mélanges monoxyde de carbone - hydrogène, dans des conditions identiques de pression, température et débit, sont indiqués dans le tableau ci-dessous.

TABLEAU IV

| Technique de préparation | $CH_3OH$  p.p.m. |
|---|---|
| Imprégnation | 148 |
| Mélange mécanique | 0 |

Le catalyseur préparé par mélange mécanique est inactif pour la synthèse du méthanol.

0156691

## REVENDICATIONS

1. Méthode de préparation de catalyseurs de synthèse du méthanol à base de cuivre associé à des supports minéraux, choisis parmi les oxydes des métaux de la famille des alcalino-terreux, des lanthanides et des actinides, caractérisée en ce que le cuivre est introduit par l'intermédiaire d'un précurseur du type formiate de cuivre par imprégnation du support par une solution de formiate de cuivre, puis soumission de la masse catalytique à une thermodécomposition sous courant d'hydrogène à une température comprise entre 250 et 400°C, pendant une durée supérieure à une heure.

2. Méthode de préparation de catalyseurs de synthèse du méthanol à base de cuivre associé à un support minéral selon la revendication 1, caractérisée en ce que le précurseur est dissous dans l'eau, l'oxyde métallique étant ajouté en suspension dans cette solution, après une durée d'imprégnation comprise entre 1 à 15 minutes, la solution est évaporée sous vide, à température comprise entre 60 et 95°C, jusqu'à siccité, et le solide est soumis à un broyage.

3. Méthode de préparation de catalyseurs de synthèse du méthanol à base de cuivre associé à un support minéral selon la revendication 1, caractérisée en ce que la masse catalytique obtenue par broyage est thermodécomposée sous courant d'hydrogène à une température comprise entre 250 et 400°C, pendant une durée supérieure à une heure, la vitesse volumétrique de l'hydrogène étant comprise entre environ 5.000 et 50.000 $h^{-1}$.

4. Méthode de préparation de catalyseurs de synthèse du méthanol à base de cuivre associé à un support minéral selon la revendication 3, caractérisée en ce que la masse catalytique est thermodécomposée sous courant d'hydrogène à une température de 300°C, après une montée en température de 1°C $mn^{-1}$, la vitesse volumétrique de l'hydrogène étant de l'ordre de 12.500 $h^{-1}$.

5. Catalyseur de synthèse du méthanol à base de cuivre associé à un support minéral, obtenu selon la méthode d'une des revendications 1 à 4, caractérisé en ce que le catalyseur réduit contient entre 10 et 50 % de cuivre, de préférence entre environ 15 et 25 %, associé à l'oxyde de magnésium (MgO).

6. Catalyseur de synthèse du méthanol à base de cuivre associé à un support minéral, obtenu selon la méthode d'une des revendications 1 à 4, caractérisé en ce que le catalyseur réduit contient entre 0,5 et 10 % de cuivre, de préférence entre 1 et 5 %,

0156691

associé à la thorine ($ThO_2$).

7. Application des catalyseurs obtenus selon une quelconque des revendications 1 à 6, à la synthèse du méthanol à partir de mélange monoxyde de carbone-hydrogène.

Office européen
des brevets

**RAPPORT DE RECHERCHE EUROPEENNE**

EP 85 40 0354

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | US-A-3 284 370 (A.F. CLIFFORD) | 1,2,5-7 | B 01 J 23/76<br>C 07 C 29/15 |
| A | FR-A- 938 605 (UNIVERSAL OIL PRODUCTS) | 1,7 | |
| A | US-A-2 338 805 (H. DREYFUS) | 1,3,4,7 | |
| A | GB-A- 308 181 (I.G. FARBENINDUSTRIE) | 1,3,7 | |

| | | | DOMAINES TECHNIQUES RECHERCHES (Int. Cl. ³) |
|---|---|---|---|
| | | | B 01 J<br>C 07 C |

Le présent rapport de recherche a été établi pour toutes les revendications

| Lieu de la recherche | Date d'achèvement de la recherche | Examinateur |
|---|---|---|
| LA HAYE | 06-06-1985 | THION M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82